# EUROPEAN PATENT SPECIFICATION

(11) **EP 4 534 983 B1**
(45) Date of publication and mention of the grant of the patent: **27.05.2026**
(21) Application number: 25159247.3
(22) Date of filing: 01.04.2021
(51) Int. Cl.: G01N 35/08, G01N 21/05, G01N 21/78, G01N 37/00, B01L 3/00, G01N 33/543, G01N 21/03, G01N 21/77

(54) **TEST CHIP AND METHOD FOR MANUFACTURING THE SAME**
TESTCHIP UND VERFAHREN ZU SEINER HERSTELLUNG
PUCE DE TEST ET SON PROCÉDÉ DE FABRICATION

(30) Priority: 28.04.2020 JP 2020079845; 07.01.2021 JP 2021001630
(43) Date of publication of application: 09.04.2025
(62) Divisional of application: 21797891.5
(73) Proprietor: Dexerials Corporation, Shimotsuke-shi, Tochigi 323-0194 (JP)
(72) Inventor: MONJU, Takuya, Shimotsuke-shi Tochigi 323-0194 (JP); SUMA, Motoki, Shimotsuke-shi Tochigi 323-0194 (JP)
(74) Representative: Scott, Stephen John

(56) References cited:
- EP-A1- 2 589 435
- US-A1- 2012 198 684
- US-A1- 2013 078 711
- US-B2- 8 911 683

## Description

### TECHNICAL FIELD

This disclosure relates to a test chip and a method for manufacturing the same.

### BACKGROUND

There is an idea that diagnosis carried out in the immediate vicinity of patients or the like (on-the-spot diagnosis) is highly important, and such an idea is being generally accepted in clinical practice. Based on such idea, test chips have been developed that enable practical analysis by providing a sheet-shaped substrate with micro-sized passages and reaction spots.

One example of such test chip includes a mechanism wherein, when a test liquid containing a target substance such as antigen is introduced, the test liquid flows through a passage and reacts with a labeling medium, such as antibody that has been charged in advance, thereby allowing the presence of the target substance to be confirmed by means of color development (revelation). A typical example is pregnancy test drug.

As the test chip described above, there has been reported a microfluidic device in which a paper is used as a base material and a passage or a reaction spot is formed on the paper using a wax printer or an inkjet printer (Whiteside et al., Analytical Chemistry, Vol. 82, No. 1, January 1, 2010 (NPL 1)). Such devices are also referred to as "µ-PADs (microfluidic paper-based analytical devices)" and have many advantages, such as (1) low cost, (2) pumpless, (3) no requirement for large-scale equipment, and (4) easy disposal. Research for improvement in such µ-PADs is being promoted globally.

For example, WO 2012/160857 A (PTL 1) discloses that the test chip can be manufactured easily and at low cost, by printing the outer edge of the passage or reaction spot as described above on a paper with ultraviolet curable ink, and curing the ink by irradiating with ultraviolet rays. EP 2 589 435 A1 (PTL 2) discloses a three-dimensional microfluidic device in which a hydrophobic substance is caused to permeate a porous material in such a way as to delimit a channel. US 2013/078711 A1 (PTL 3) discloses a microfluidic valve system in which a hydrophobic material is caused to permeate the thickness of a hydrophilic media to define a hydrophobic channel that separates a hydrophilic transfer region and a hydrophilic acceptor region. US 2012/198684 A1 (PTL 4) discloses methods for patterning hydrophobic regions on hydrophilic substrates using a wax material. US 8 911 683 B2 (PTL 5) discloses a microfluidic device that includes at least one chamber accessible by microfluidic channels, wherein the surfaces of the channels and the chambers are patterned and comprise hydrophilic and hydrophobic areas.

### Patent Literature

PTL 1: WO 2012/160857 A
PTL 2: EP 2 589 435 A1
PTL 3: US 2013/078711 A1
PTL 4: US 2012/198684 A1
PTL 5: US 8 911 683 B2

### Non-patent Literature

NPL 1: Whiteside et al., Analytical Chemistry, Vol. 82, No. 1, January 1, 2010

### SUMMARY

### (Technical Problem)

However, in the conventional test chip including the technology disclosed in PTL 1 described above, unevenness in color development (color development) tends to occur and cause fluctuation in the inspection results (insufficient reproducibility). For example, as the color development occurs near the end of the detection area, there is a problem that visual observation is difficult. Such a problem may adversely affect the diagnosis of important diseases, etc. Thus, the conventional test chip has a room for improvement in terms of suppressing uneven color development.

Further, for the above-mentioned test chips such as µ-PADs, in addition to confirmation of the presence or absence of the substance to be detected, it is desirable to realize the quantification thereof. In this regard, if it is unevenness of color development (color development) as described above can be suppressed, then it is possible to realize quantification by specifying the area of the color-developed part in the test chip and applying the pixel analysis technology. From this viewpoint also, it is desired to suppress uneven color development.

The task underlying the present disclosure is to solve the above-mentioned problems in the conventional device and to achieve the objects as follows. That is, it is an object of the present disclosure to provide a test chip wherein the target substance contained in the test liquid is reacted with the pre-charged labeling medium for confirming the presence of the target substance by the reaction between, and wherein uneven color development is significantly suppressed. It is another object of the present disclosure to provide a method for manufacturing the above-mentioned test chip, with which the test chip can be manufactured easily, high accurately and at low cost.

### (Solution to Problem)

In the course of solving the above problem, the inventors found for the first time that the problem in the conventional test chip of the occurrence of color development near the end of the detection area is caused by the momentum of the liquid flow reaching the detection area.

Further, the inventors conducted extensive studies and found that color development could be induced near the center of the detection area if the liquid passage is optimized so that the liquid can reach the detection area from the thickness direction of the sheet, which led to the completion of the present disclosure.

The means for achieving the above object is summarized in the following clauses.

<1> A sheet-shaped test chip comprising:
   a first layer on a front surface side and a second layer on a back surface side;
   wherein the first layer and the second layer are adjacent to each other;
   wherein one of the first layer and the second layer has a liquid receiving section A;
   wherein the first layer has at least a detection confirmation section B;
   wherein the second layer has at least a liquid flow section D adjacent to the detection confirmation section B, and a liquid passage E connected to the liquid flow section D;
   wherein, in case where the liquid receiving section A is provided in the first layer, the liquid receiving section A is spaced from the detection confirmation section B;
   wherein, when a sample test liquid is dropped into the liquid receiving section A, the test liquid passes through the liquid receiving section A, the liquid passage E, and the liquid flow section D in the stated order, by means of capillary action, and flows to the detection confirmation section B; and
   wherein the liquid flow section D has an annular structure formed with a liquid non-flow section D' therein.
<2> The test chip according to <1>, wherein the second layer is provided with a plurality of the liquid passages E.
<3> The test chip according to <2>, wherein at least two of the liquid passages E are connected to the liquid flow section D so as to face each other.
<4> The test chip according to <2> or <3>, wherein at least two of the liquid passages E have substantially the same shape.
<5> The test chip according to any one of <1> to <4>, wherein:
   the first layer comprises the liquid receiving section A that is spaced from the detection confirmation section B, the second layer comprises the liquid flow section C adjacent to the liquid receiving section A; and
   the test chip is configured such that, when the sample test liquid is dropped into the liquid receiving section A, the test liquid passes through the liquid receiving section A, the liquid flow section C, the liquid passage E, and the liquid flow section D in this order, due to a capillary action and flows to the detection confirmation section B.
<6> The test chip according to any one of <1> to <4>, wherein:
   the first layer comprises the liquid receiving section A that is spaced from the detection confirmation section B, and the liquid passage F that is connected to the liquid receiving section A; and
   the test chip is configured so that, when the test liquid is dropped into the liquid receiving section A, the test liquid passes through the liquid receiving portion A, the liquid passage F, the liquid passage E, and the liquid flow section D in this order, due to the capillary action and flows to the detection confirmation section B.
<7> The test chip according to any one of <1> to <4>, wherein the second layer comprises the liquid receiving portion A.
<8> The test chip according to any one of <1> to <7>, wherein:
   the liquid receiving section A, any liquid flow section C, any liquid passage F, the liquid passage E, the liquid flow section D, and the detection confirmation section B, are made of a material M that permits flow of the test liquid by means of capillary action; and
   the part other than the material M is made of a material M' in which the material M is impregnated with the hydrophobic material that prohibits flow of the test liquid.
<9> The test chip according to <8>, wherein the material M is a filter paper.
<10> The test chip according to <8> or <9> wherein, in the material M', an impregnation rate of the hydrophobic material into the material M is 14% or more and 32% or less.
<11> The test chip according to any one of <1> to <10>, wherein a ratio of the thickness of the second layer to the thickness of the first layer (thickness of the second layer / thickness of the first layer) is 0.56 or more and 2.2 or less.
<12> The test chip according to any one of <1> to <11>, being provided with a medium that reacts with a substance to be detected and/or a labeling medium adapted to cause a color reaction due to the substance to be detected so that a color development reaction due to a substance to be detected takes place in the detection confirmation section B.
<13> A method for manufacturing a test chip of any one of <1> to <12>, comprising:
   a film formation step wherein a hydrophobic material is used for forming a first hydrophobic film on a first substrate, and a second hydrophobic film on a second substrate;
   a first printing step wherein the first hydrophobic film on the first substrate is used for carrying out printing on a first sacrificial substrate, so as to be of an inverted pattern of a first layer of the test chip;
   a second printing process wherein the second hydrophobic film on the second substrate is used for carrying out printing on a second sacrificial substrate, so as to be of an inverted pattern of the second layer of the test chip;
   a first transfer step wherein the first hydrophobic film after the first printing step is transferred onto one surface of a single sheet-like material and impregnated into the sheet-like material; and
   a second transfer step wherein the second hydrophobic film after the second printing step is transferred onto the other surface of the single sheet-like material and impregnated into the sheet-like material.
<14> The method for manufacturing a test chip according to <13>, wherein a ratio of the thickness of the second hydrophobic membrane to the thickness of the first hydrophobic membrane (thickness of the second hydrophobic membrane / thickness of the first hydrophobic membrane) is 0.56 or more and 2.2 or less.

### (Advantageous Effect)

According to the present disclosure, it is possible to provide a test chip wherein the target substance contained in the test liquid is reacted with the pre-charged labeling medium for confirming the presence of the target substance by the reaction between, and wherein uneven color development is significantly suppressed. Furthermore, according to the present disclosure, it is also possible to provide a method for manufacturing the above-mentioned test chip, with which the test chip can be manufactured easily, high accurately and at low cost.

### BRIEF DESCRIPTION OF THE DRAWINGS

FIG. 1A is a schematic perspective view of the test chip according to one embodiment of the present disclosure.
FIG, 1B is a schematic perspective view of the test chip of FIG. 1A.
FIG. 2 is a schematic plan view of the front surface and the back surface of the test chip of FIG. 1A.
FIG. 3 is a schematic cross-sectional view of the test chip of FIG. 1A.
FIG. 4 is the schematic exploded view of the test chip according to one embodiment of the present disclosure.
FIG. 5 is a schematic plan view of the front surface and the back surface of the test chip according to one embodiment of the present disclosure.
FIG. 6 is a schematic cross-sectional view of the test chip of FIG. 5.
FIG. 7 is a schematic plan view of the front surface and the back surface of the test chip according to one embodiment of the present disclosure.
FIG. 8 is a schematic cross-sectional view of the test chip of FIG. 7.
FIG. 9 is a schematic plan view of the front surface and the back surface of the test chip according to one embodiment of the present disclosure.
FIG. 10 is a schematic plan view of the front surface and the back surface of the test chip according to one embodiment of the present disclosure.
FIG. 11 is a schematic plan view of the front surface and the back surface of the test chip according to one embodiment of the present disclosure.
FIG. 12 is a schematic plan view of the front surface and the back surface of the test chip according to one embodiment of the present disclosure.
FIG. 13 is a schematic plan view of the front surface and the back surface of the test chip according to one embodiment of the present disclosure.
FIG. 14 is a schematic plan view of the front surface and the back surface of the test chip according to one embodiment of the present disclosure.
FIG. 15 is a schematic cross-sectional view of the test chip according to one embodiment of the present disclosure.
FIG. 16 is a schematic plan view of the front surface and the back surface of the test chip according to one embodiment of the present disclosure.

### DETAILED DESCRIPTION

Hereinafter, the present disclosure will be described in detail based on the embodiments.

### (Test chip)

According to the present disclosure, there is provided a sheet-shaped test chip as set out in the appended set of claims

Further, the test chip according to one embodiment of the present disclosure is further characterized in that, in addition to the basic features noted above, the liquid flow section D has an annular structure formed with a liquid non-flow section D' therein. The details of this feature will be described later.

The test chip according to another embodiment of the present disclosure is further characterized in that, in addition to the basic features noted above, the second layer is provided with a plurality of the liquid passages E. The details of this feature will be described later.

The test chip according to still another embodiment of the present disclosure is further characterized in that, in addition to the basic features noted above, the first layer is formed on one surface of a single sheet-like material, and the second layer is formed on the other surface of the sheet-like material. The details of this feature will be described later.

According to these test chips, it is possible to significantly suppress uneven color development.

In the test chip of the present disclosure, the liquid receiving portion A is a portion where the test liquid is dropped. Further, in the test chip of the present disclosure, the detection confirmation section B is a portion where confirmation is made as to whether or not a target substance, such as an antigen, is present in the test liquid dropped on the liquid receiving section A, by means of the presence or absence of color development. Thus, the test chip 1 of the present disclosure may be provided with a medium that reacts with the substance to be detected and / or a labeling medium adapted to cause a color reaction due to the substance to be detected. Furthermore, it is preferred that, in the test chip 1 of the present disclosure, the color development reaction due to the substance to be detected takes place in the detection confirmation section B.

More specifically, the aspects of the above-mentioned basic features common to the test chips of the present disclosure may include an aspect wherein the liquid receiving section A is provided in the first layer and the second layer has the liquid flow section C (first aspect), an aspect wherein the liquid receiving section A is provided in the first layer, and the first layer is further provided with a liquid passage F connected to the liquid receiving portion A (second aspect), and an aspect wherein the liquid receiving portion A is provided in the second layer (third aspect).

### <First aspect of the basic features>

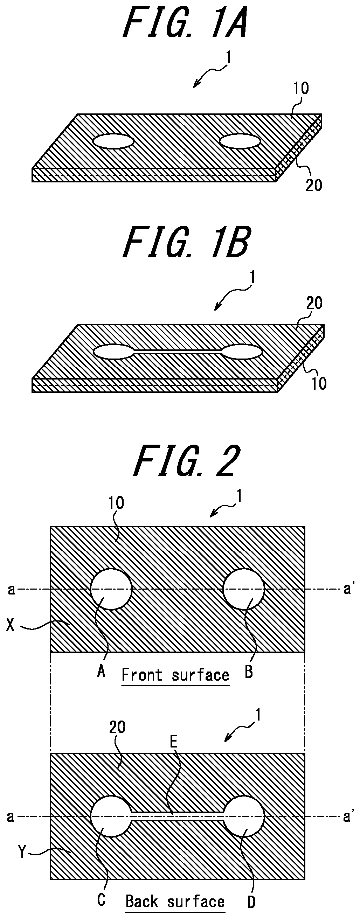

FIGS. 1A and 1B are schematic perspective views of a test chip 1 according to the first aspect, respectively. The test chip 1 includes a first layer 10 on the front surface side, that is, the surface side on which an inspector visually recognizes the inspection results during use, and a second layer 20 on the back surface side thereof. FIG. 1A is a perspective view with the front surface of the test chip 1 on the upper side, and FIG. 1B is a perspective view with the back surface of the test chip 1 on the upper side. As illustrated in FIGS. 1A and 1B, the test chip 1 is of a sheet shape. Further, the first layer 10 and the second layer 20 of the test chip 1 do not have any intervening objects and are adjacent to each other. The shape of the test chip 1 in a plan view is not particularly limited and may be appropriately selected depending on the intended purpose; for example, it may be rectangular as illustrated in FIGS. 1A and 1B, or may be circular, oval or the like.

FIG. 2 is a schematic plan view of the front surface and the back surface of the test chip 1, and the structure thereof corresponds to the test chip 1 illustrated in FIGS. 1A and 1B. As illustrated in FIG. 2, the test chip 1 is provided with a liquid receiving section A and a detection confirmation section B on the first layer 10 that is arranged on the front surface side. The liquid receiving section A and the detection confirmation section B are spaced from each other in the first layer 10 of the test chip 1.

The first layer 10 of the test chip 1 is provided with a liquid non-flow section X as a portion other than the liquid receiving section A and the detection confirmation section B. The liquid receiving section A and the detection confirmation section B are made, for example, of a material M that permits flow of the test liquid by capillary action. Further, the liquid non-flow section X is made, for example, of a material M' that prohibits (does not permit) flow of the test liquid.

As illustrated in FIG. 2, the test chip 1 is provided with a liquid flow section C, a liquid flow section D, and a liquid passage E on the second layer 20 that is arranged on the back surface side. The liquid passage E is connected to the liquid flow section D and also to the liquid flow section C. The second layer 20 of the test chip 1 is provided with a liquid non-flow section Y, as a section other than the liquid flow section C, the liquid passage E, and the liquid flow section D. The liquid flow section C, the liquid passage E, and the liquid flow section D are made of a material M that permits flow of the test liquid by capillary action, as in the case of the liquid receiving section A and the detection confirmation section B described above. Further, the liquid non-flow section Y is made of a material M' that prohibits flow of the test liquid, for example, as in the case of the liquid non-flow section X described above.

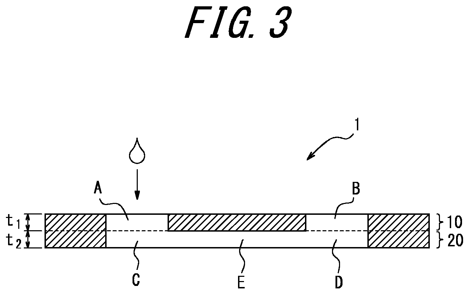

FIG. 3 is a schematic cross-sectional view, with the test chip 1 of FIG. 2 being cut along the line a-a. As illustrated in FIG. 3, in the test chip 1 according to the first aspect, the liquid receiving section A of the first layer 10 and the liquid flow section C of the second layer 20 are adjacent to each other, and the detection confirmation section B of the first layer 10 and the liquid flow section D of the second layer 20 are adjacent to each other. That is, in the test chip 1 according to the first aspect, the liquid receiving section A, the liquid flow section C, the liquid passage E, the liquid flow section D, and the detection confirmation section B are adjacent to each other or connected to each other in this order. In other words, the test chip 1 according to the first aspect is configured so that, the test liquid dropped into the liquid receiving section A is caused to flow eventually to the detection confirmation section B by the capillary action, via the liquid receiving section A, the liquid flow section C, the liquid passage E and the liquid flow section D in this order.

Here, in the conventional test chip, since the liquid receiving section and the detection confirmation section (detection area) are simply connected by a passage on the base material, when the test liquid is dropped into the liquid receiving section, there is a problem of unevenness of the color development such that the color development is distributed unevenly near the end of the detection area (particularly near the end that is remote from the liquid receiving section), due to the momentum of the liquid flow flowing along the surface direction of the material and reaching the detection area. On the other hand, in the test chip 1 as described above, since the test chip 1 has the above-mentioned configuration, the test liquid dropped into the liquid receiving section A is flows eventually in the thickness direction of the test chip 1 (the direction from the liquid flow section D to the detection confirmation section), and reaches the detection confirmation section B. Thus, in the test chip 1 according to the first aspect, the color development site can be maintained near the center of the detection confirmation section B, thereby significantly suppressing unevenness of the color development.

As described above, the liquid receiving section A and the detection confirmation section B in the first layer 10, and the liquid flow section C, the liquid flow section D, and the liquid passage E in the second layer 20 are made, for example, of the material M that permits flow of the test liquid due to the capillary action. On the other hand, the liquid non-flowing section X in the first layer 10 and the liquid non-flowing section Y in the second layer 20 are made, for example, of the material M' in which the material M is impregnated with a hydrophobic material, and flow of the test liquid is prohibited.

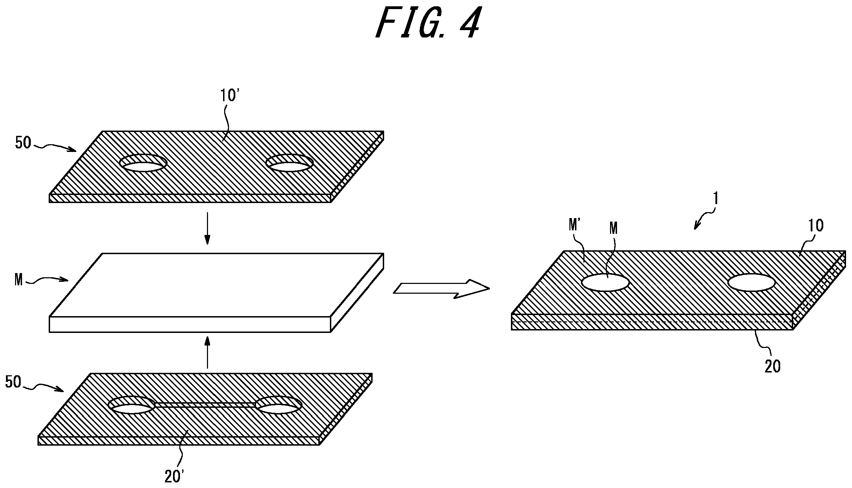

FIG. 4 is a schematically exploded view of the test chip 1 of FIGS. 1A and 1B. As illustrated on the left side of FIG. 4, in a schematic sense, the test chip 1 is comprised of the above material M, a pre-first layer 10' made of a hydrophobic material 50 having the pattern of the first layer 10, and a pre-second layer 20' made of a hydrophobic material 50 having a pattern of a second layer 20. Further, as illustrated on the left side of FIG. 4, the test chip 1 has a structure in which the pre-first layer 10'and the pre-second layer 20' are impregnated with the material M from both sides. Then, as illustrated on the right side of FIG. 4, from the viewpoint of material, the test chip 1 is comprised, for example, of the material M and the material M' in which the material M is impregnated with the hydrophobic material 50.

The material M is not particularly limited as long as it causes a capillary action, and may include paper, such as filter paper, non-woven fabric, nitrocellulose, polypropylene, etc. Among these, from the viewpoint that a test chip can be produced more easily and at low cost, the material M is preferably a filter paper. Furthermore, the thickness and the basis weight (density) of the material M may be appropriately selected in consideration of the viscosity of the test liquid, etc.

The hydrophobic material is not particularly limited as long as it can be impregnated into the material M and inhibits the capillary action in the material M, and examples thereof may include wax or a composition containing the same. Further, from the viewpoint of easy production, the hydrophobic material preferably has a melting point of 90°C, or lower.

In the test chip described above, it is preferred that the material M' of the liquid non-flow section X on the front surface side is colored so that the inspector can easily observe the liquid receiving section A and the detection confirmation section B on the front surface side. On the other hand, the material M' of the liquid non-circulation portion Y on the back surface side of the test chip may be colored, white or transparent, or may not be colored.

The coloring of the material M' may be carried out for example, by impregnating the material M with a colorant in addition to the hydrophobic material. Such a colorant is preferably hydrophobic, and examples thereof may include pigments such as carbon black (black pigment). Further, it is preferred to select a colorant that does not adversely affect the reagent used for the test chip.

The shape of the liquid receiving portion A in a plan view is not particularly limited and may be appropriately selected depending on the intended purpose; for example, it may be circular as illustrated in FIG. 2, or may be elliptical, rectangular, etc.

The shape of the detection confirmation section B in a plan view is not particularly limited and may be appropriately selected depending on the intended purpose; it may be circular as illustrated in FIG. 2, or may be elliptical, rectangular or, etc.

The shape of the liquid flow section C in a plan view is not particularly limited and may be appropriately selected depending on the intended purpose; preferably, it is the same shape as the liquid receiving section A. Further, it is preferred that the liquid flow section C has a shape that substantially matches the liquid receiving section A in the plan view of the test chip.

The shape of the liquid flow section D in a plan view is not particularly limited and may be appropriately selected depending on the intended purpose; preferably, it is the same shape as the detection confirmation section B. Further, it is preferred that the liquid flow section D has a shape that substantially matches the detection confirmation section B in the plan view of the test chip.

The thickness of the test chip according to the present disclosure is not particularly limited, and may be 100 to 300 µm, for example.

Although not illustrated, the test chip of the present disclosure may have a plurality of sets of combination of the detection confirmation section B, the liquid flow section D, and the liquid passage E. In this case, the presence or absence of a plurality of substances to be detected can be confirmed at the same time, through a single inspection.

### <Second aspect of the basic features>

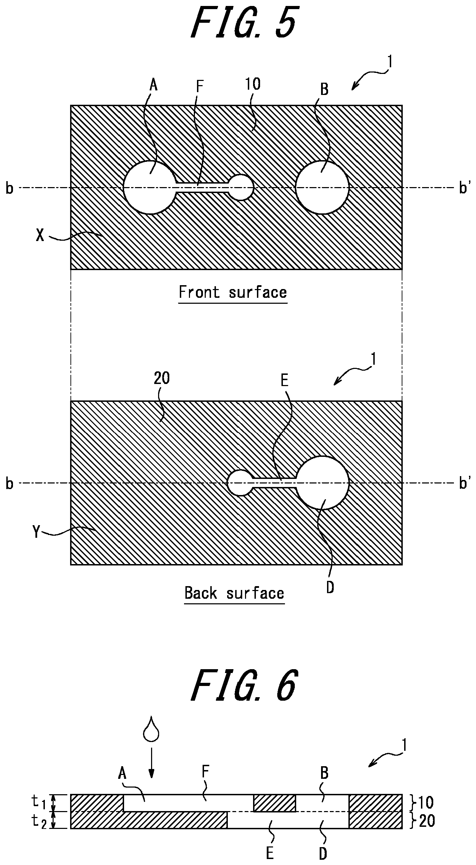

FIG. 5 is a schematic plan view of the front surface and the back surface of the test chip 1 according to the second aspect. As illustrated in FIG. 5, the test chip 1 is provided with a liquid receiving section A and a detection confirmation section B on the first layer 10 arranged on the front surface side. The liquid receiving section A and the detection confirmation section B are spaced from each other in a first layer 10 of the test chip 1. Further, the test chip 1 has a liquid passage F connected to the liquid receiving portion A in the first layer 10 arranged on the front surface side.

Further, the first layer 10 of the test chip 1 is provided with a liquid non-flow section X, as a portion other than the liquid receiving section A, the detection confirmation section B, and the liquid passage F. The liquid receiving section A, the detection confirmation section B, and the liquid passage F are made, for example, of a material M that permits flow of the test liquid by the capillary action. Further, the liquid non-flow section X is made, for example, of a material M' that prohibits flow of the test liquid.

As illustrated in FIG. 5, the test chip 1 is provided with a liquid flow section D and a liquid passage E in a second layer 20 arranged on the back surface side. The liquid passage E is connected to the liquid flow section D. Further, the second layer 20 of the test chip 1 is provided with a liquid non-flow section Y, as a portion other than the liquid passage E and the liquid flow section D. The liquid passage E and the liquid passage D are made, for example, of the material M that permits flow of the test liquid is expressed by the capillary action, as in the liquid receiving section A, the detection confirmation section B, and the liquid passage F described above. Further, the liquid non-flow section Y is made, for example, of a material M' that prohibits flow of the test liquid, as in the liquid non-flow section X described above.

FIG. 6 is a schematic cross-sectional view, with the test chip 1 of FIG. 5 being cut along the line b-b'. As illustrated in FIG. 6, in the test chip 1 according to the second aspect, the detection confirmation section B of the first layer 10 and the liquid flow section D of the second layer 20 are adjacent to each other, and the liquid passage F of the first layer 10 and the liquid passage E of the second layer 20 are connected with each other. That is, in the test chip 1 according to the second aspect, the liquid receiving section A, the liquid passage F, the liquid passage E, the liquid flow section D, and the detection confirmation section B are adjacent to each other or connected to each other in this order. In other words, the test chip 1 according to the second aspect is configured such that, when the test liquid is dropped on the liquid receiving portion A, the test liquid passes the liquid receiving portion A, the liquid passage F, and the liquid passage E, and the liquid flow section D in this order, due to the capillary action, and eventually flows to the detection confirmation section B. Thus, in the test chip 1 according to the second aspect, as in the first aspect, the test liquid dropped on the liquid receiving portion A is permitted to flow in the thickness direction of the test chip 1 (the direction from the liquid flow section D toward the detection confirmation section B) and to eventually reach the detection confirmation section B. Therefore, also in the test chip 1 according to the second aspect, the color development site can be maintained near the center of the detection confirmation section B, thereby significantly suppressing unevenness of the color development.

As described above, the liquid receiving section A, the detection confirmation section B and the liquid passage F in the first layer 10, and the liquid flow section D and the liquid passage E in the second layer 20 are made, for example, of a material M that permits flow of the test liquid due to the capillary action. On the other hand, the liquid non-flow section X in the first layer 10 and the liquid non-flow section Y in the second layer 20 are made, for example, of the material M' in which the material M is impregnated with a hydrophobic material, which prohibits the flow of the test liquid.

In the test chip described above, it is preferred that the material M' of the liquid non-flow section X on the front surface side is colored so that the inspector can easily observe the liquid receiving section A and the detection confirmation section B on the front surface side. On the other hand, the material M' of the liquid non-circulation portion Y on the back surface side of the test chip may be colored, white or transparent, or may not be colored.

Other than the above, description of the features of the second aspect common to the first aspect is omitted.

### <Third aspect of the basic features>

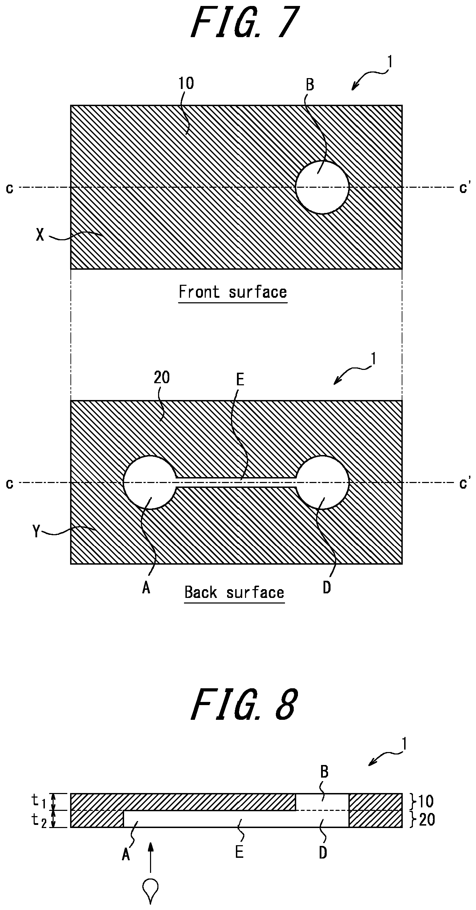

FIG. 7 is a schematic plan view of the front surface and the back surface of the test chip 1 according to the third aspect. As illustrated in FIG. 7, the test chip 1 is provided with the detection confirmation section B that is arranged in the first layer 10 arranged on the front surface side, but is not provided with the liquid receiving section A.

Further, the first layer 10 of the test chip 1 is provided with a liquid non-flow section X as a portion other than the detection confirmation section B. The detection confirmation section B is made, for example, of a material M that permits flow of the test liquid by the capillary action. Further, the liquid non-flow section X is made, for example, of a material M' that prohibits flow of the test liquid.

Further, as illustrated in FIG. 7, the test chip 1 is provided with a liquid receiving section A, a liquid flow section D, and a liquid passage E on a second layer 20 arranged on the back surface side. The liquid passage E is connected to the liquid flow section D and is also connected to the liquid receiving section A. Further, the second layer 20 of the test chip 1 is provided with a liquid non-flow section Y, as a portion other than the liquid receiving section A, the liquid passage E, and the liquid flow section D. The liquid receiving section A, the liquid passage E, and the liquid flowing section D are made, for example, of a material M that permits flow of the test liquid by the capillary action, as in the detection confirmation section B described above. Further, the liquid non-flow section Y is made, for example, of a material M' that prohibits flow of the test liquid, as in the liquid non-flow section X described above.

FIG. 8 is a schematic cross-sectional view, with the test chip 1 of FIG. 7 being cut along the line c-c. As illustrated in FIG. 8, in the test chip 1 according to the third aspect, the detection confirmation section B of the first layer 10 and the liquid flow section D of the second layer 20 are adjacent to each other. That is, in the test chip 1 according to the third aspect, the liquid receiving section A, the liquid passage E, the liquid flow section D, and the detection confirmation section B are adjacent to each other or connected with each other in this order. In other words, the test chip 1 according to the third aspect is configured such that the test liquid dropped into the liquid receiving portion A is caused to flow by the capillary action through the liquid receiving portion A, the liquid passage E, and the liquid flow section D in this order, and to eventually reach the detection confirmation section B. Thus, in the test chip 1 according to the third aspect, as in the first aspect, the test liquid dropped on the liquid receiving portion A is permitted to flow in the thickness direction of the test chip 1 (the direction from the liquid flow section D toward the detection confirmation section B) and to eventually reach the detection confirmation section B. Therefore, also in the test chip 1 according to the third aspect, the color development site can be maintained near the center of the detection confirmation section B, thereby significantly suppressing unevenness of the color development.

As described above, the detection confirmation section B and the liquid passage F in the first layer 10, and the liquid flow section D and the liquid passage E in the second layer 20 are made, for example, of a material M that permits flow of the test liquid due to the capillary action. On the other hand, the liquid non-flow section X in the first layer 10 and the liquid non-flow section Y in the second layer 20 are made, for example, of a material M' in which the material M is impregnated with a hydrophobic material, which prohibits flow of the test liquid.

In the test chip described above, it is preferred that the material M' of the liquid non-flow section X on the front surface side and the liquid non-flow section Y on the back surface side is colored so that the inspector can easily observe the liquid receiving portion A and the detection confirmation portion B on the front surface side.

The test chip according to the third aspect is particularly useful, for example, when it is desired to arrange the liquid receiving section A and the detection confirmation section B on different surfaces from the viewpoint of fail-safe.

Other than the above, description of the features of the second aspect common to the first aspect is omitted.

Further, the test chip according to the present disclosure may appropriately include the features described below. Hereinafter, the description will be made with reference to the features according to the first aspect as the basic feature, though any of the features may be applied to the case of the second aspect and the third aspect.

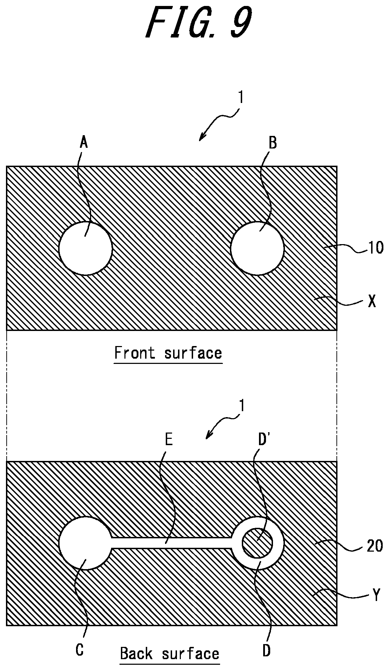

FIG. 9 is a schematic plan view of the front surface and the back surface of the test chip 1 according to one embodiment. The test chip 1 illustrated in FIG. 9 is essentially same as that of FIG. 2, except the features that the liquid flow section D provided in the second layer 20 on the back surface side has an annular structure, and formed therein with a liquid non-flow section D'. In the test chip 1, due to the presence of the liquid non-flow section D', when the test liquid advances from the liquid flow section D toward the detection confirmation section B, there can be induced a liquid flow from the outside to the center in the detection confirmation section B. Therefore, the color development can be further effectively maintained near the center of the detection confirmation section B, so that unevenness of the color development can be suppressed even more significantly.

In the test chip 1 as described above, the liquid flow section D having an annular structure may have any contour shape such as circle, ellipse, or rectangle, though it is preferred that the contour shape substantially matches the detection confirmation portion B in the plan view of the test chip. Further, it is preferred that the liquid non-flow section D' in the plan view of the test chip has a shape obtained by scale-reduction of the contour shape of the liquid flow section D. Further, it is preferred that the liquid non-flow section D' is colored in white, transparent, or uncolored so as not to disturb confirmation of the color development in the detection confirmation section B.

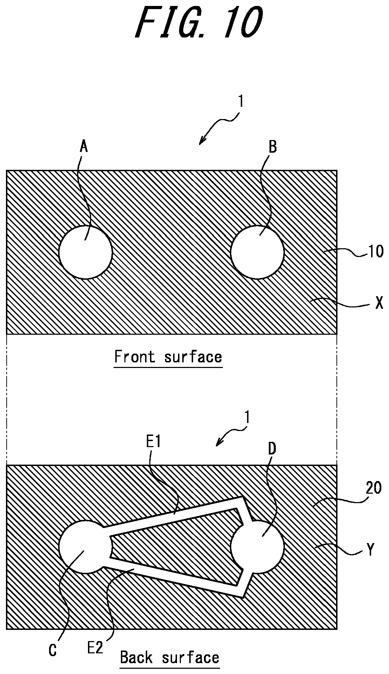

FIG. 10 is a schematic plan view of the front surface and the back surface of the test chip 1 of another embodiment. The test chip 1 illustrated in FIG. 10 is generally the same as that of FIG. 2, except that the second layer 20 arranged on the back surface side has a plurality of liquid passages E (two in FIG. 10, i.e., E1 and E2). In the test chip 1, when the test liquid travels from the liquid flow section D toward the detection confirmation section B, due to the presence of a plurality of liquid passages E, the momentum of the liquid flow from each liquid passage E is cancelled so as to promote the liquid flow in the thickness direction of the test chip 1. Therefore, the color development site can be further effectively maintained near the center of the detection confirmation section B, so as to even more significantly suppress the unevenness of the color development.

Regarding the abovementioned feature, in the case of the test chip according to the second aspect, a plurality of liquid passages F may be provided as many as the number of liquid passages E, and the plurality of liquid passages F of the first layer 10 and the plurality of liquid passages E of the second layer 20 may be connected to each other, respectively.

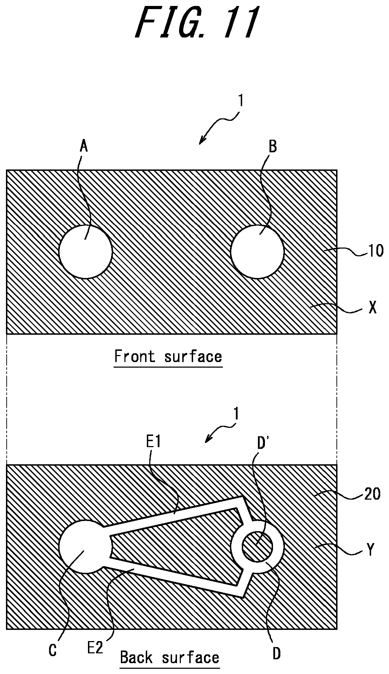

FIG. 11 is a schematic plan view of the front surface and the back surface of the test chip 1 of another embodiment. The test chip 1 illustrated in FIG. 11 is a combination of the features illustrated in FIG. 9 and the features illustrated in FIG. 10. With such a test chip 1 also, uneven color development can be suppressed even more significantly.

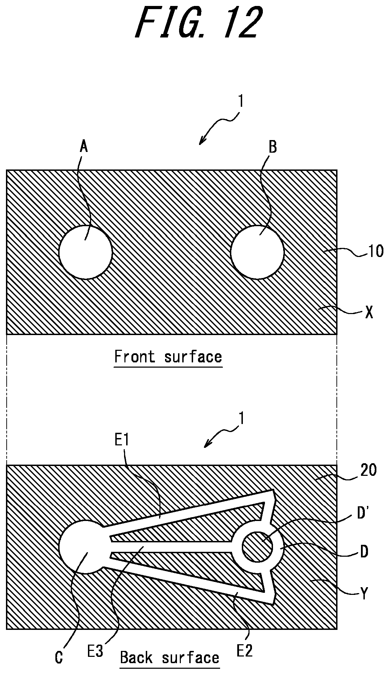

FIG. 12 is a schematic plan view of the front surface and the back surface of the test chip 1 of another embodiment. The test chip 1 illustrated in FIG. 12 is generally the same as that of FIG. 11, except that the second layer 20 arranged on the back surface side has three liquid passages E (E3 in addition to E1 and E2). In this instance, in the test chip 1 illustrated in FIG. 12, the three liquid passages E are connected to the liquid flow section D so as to face each other. With such a test chip 1 also, uneven color development can be suppressed even more significantly.

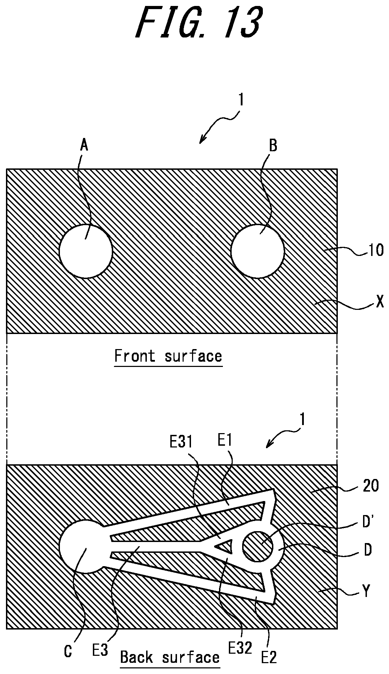

FIG. 13 is a schematic plan view of the front surface and the back surface of the test chip 1 of another embodiment. The test chip 1 illustrated in FIG. 13 is substantially the same as FIG. 12 except that the liquid passage E3 is branched into two (E31 and E32) and is connected to the liquid flow section D. With such a test chip 1 also, uneven color development can be suppressed even more significantly.

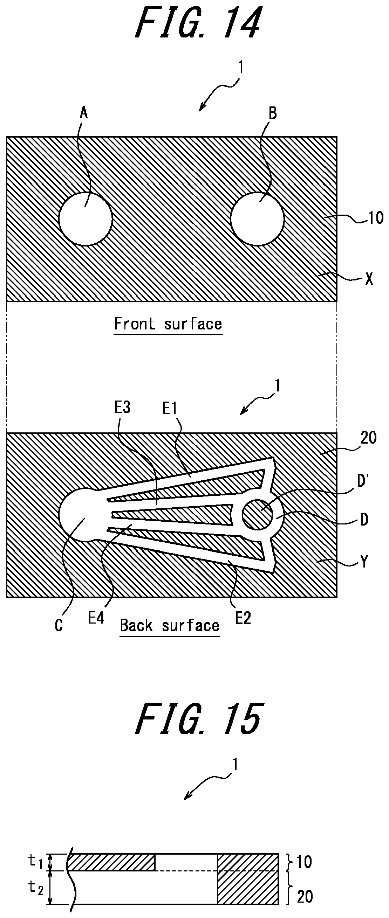

FIG. 14 is a schematic plan view of the front surface and the back surface of the test chip 1 of another embodiment. The test chip 1 illustrated in FIG. 14 is substantially the same as FIG. 11 except that it has two liquid passages E (E3 and E4) in addition to the liquid passages E1 and the liquid passage E2. In this instance, in the test chip 1 illustrated in FIG. 14, the four liquid passages E are connected to the liquid flow section D so as to face each other. With such a test chip 1 also, uneven color development can be suppressed even more significantly.

In the test chip 1 as described above, from the viewpoint of suppressing an increase in the amount of test liquid required for inspection, the number of the liquid passages E (and the liquid passages F in the case of the second aspect) is preferably four or less, more preferably three or less, and further preferably two or less. Further, the number of connection(s) of the liquid passages E to the liquid flow section D is preferably four or less, more preferably three or less, and further preferably two or less.

Further, in the test chip 1 as described above, as illustrated in FIGS. 10 to 14, it is preferred that at least two of the plurality of liquid passages E are connected to the liquid flow section D so as to face each other. By this, when the test liquid advances from the liquid flow section D toward the detection confirmation section B, it is possible to concentrate the liquid flow from each liquid passage E near the center of the liquid flow section D, and to promote the flow of liquid in the thickness direction of the test chip 1. Therefore, the color development site can be further effectively maintained near the center of the detection confirmation section B to even more significantly suppress unevenness of the color development.

Further, in the test chip 1 as described above, as illustrated in FIGS. 10 to 14, it is preferred that at least two of the plurality of liquid passages E have substantially the same shape. In this case, since the liquid flowing through the liquid passage E can reach the liquid flow section D and the detection confirmation section B almost simultaneously, it is possible to avoid the drift and further significantly suppress the unevenness of the color development.

When the above-mentioned test chip 1 is comprised of the material M (the material that permits flow of the test liquid by capillary action) and the material M' (the material in which the material M is impregnated with the hydrophobic material to prohibit flow of the test liquid), it is preferred for the material M' that the impregnation rate of the hydrophobic material into the material M is preferably in the range of 14% or more and 32% or less. By manufacturing the test chip so that the impregnation rate is 14% or more, the passage wall surface (interface between the material M and the material M') of the test liquid becomes sufficiently uniform, and the flow of the test liquid from the liquid receiving section A to the detection confirmation section B can be made smoother. Further, by manufacturing the test chip so that the impregnation rate is 32% or less, it is possible to sufficiently avoid the problem such as blockage when impregnating the material M with the hydrophobic material are sufficiently avoided, and more reliably obtain a test chip with a desired passage structure.

The above-mentioned impregnation rate refers to the impregnation rate of the material M' in the region of the test chip 1 that is made of that material M' over the entire thickness direction.

The impregnation rate may be regarded as 100% for the material M' which is obtained by immersing the material M in a hydrophobic material that is heated (e.g., heated to 120°C) to have a sufficiently low viscosity, and maintaining this temperature for a sufficient time (e.g., for 3 minutes). More specifically, the impregnation rate may be determined by the method described hereinafter with reference to the examples.

The impregnation rate may be adjusted, for example, by regulating the amount of the hydrophobic material to be impregnated (the thickness of the hydrophobic film, etc.).

In the test chip 1 as described above, it is preferred that the ratio of the thickness (t2) of the second layer 20 to the thickness (t1) of the first layer 10 (the thickness of the second layer / the thickness of the first layer), i.e., the ratio t2/t1 is 0.56 or more and 2.2 or less. By manufacturing the test chip so that the ratio t2/t1 is 0.56 or more and 2.2 or less, a desired passage structure can be more reliably formed in the obtained test chip. In particular, when the test chip is manufactured by the test chip manufacturing method described later, if the test chip is manufactured so that the ratio t2/t1 is 0.56 or more and 2.2 or less, it is possible to sufficiently avoid problems such as blockage when impregnating the sheet-like material with the hydrophobic material, and effectively improve the speed and/or speed stability of the liquid flow from the liquid receiving section A to the detection confirmation section B. From the same viewpoint, the ratio t2/t1 is preferably more than 1.0, i.e., the thickness t2 of the second layer 20 is larger than the thickness t1 of the first layer 10 as illustrated in FIG. 15, more preferably 1.3 or more, and further preferably 1.8 or more. It is noted that the ratio t2/t1 is not particularly limited, and may be 3.0 or less.

The above-mentioned test chip 1 may be manufactured, for example, by providing a predetermined portion (detection confirmation section B, etc.) on a sheet-shaped material to form the first layer 10, and a predetermined portion (liquid flow section D, etc.) on another sheet-shaped material to form the second layer 20, and then laminating these two sheet-like materials. Alternatively, the above-mentioned test chip 1 may also be manufactured by providing a predetermined portion on a part of a single sheet-like material to form the first layer 10, and a predetermined portion on another part of the single sheet-like material to form the second layer 20, and folding the single sheet-like material which positioning the first layer and the second layer. However, it is preferred that the test chip 1 according to of the present embodiment is manufactured by forming a first layer on one surface side of a single sheet-like material and forming a second layer on the other surface side. The test chip of the present embodiment, in which the first layer and the second layer are formed on both sides of one sheet-like material, provides various advantages, such as (1) the labor and cost of lamination process (or folding process) can be saved; (2) the flow of the test liquid due to the capillary action between the first layer and the second layer is ensured; and (3) no jigs or the like for holding the laminated body (or the folded body) of the sheet-like material are required so that the disposal becomes easy.

The test chip 1 in which the first layer and the second layer are formed on both sides of a single sheet-like material may be manufactured, for example, by the method for manufacturing a test chip described below.

### (Method for manufacturing a test chip)

According to one embodiment of the present disclosure, there is provided a method for manufacturing a test chip as set out in the appended set of claims.

With such manufacturing method, it is possible to manufacture the above-described test chip easily, with high accuracy and at low cost.

### <Film forming step>

In the film forming step, a hydrophobic material is used to form a first hydrophobic film on a first substrate and a second hydrophobic film on a second substrate.

A colorant may be added to the hydrophobic material. Further, the hydrophobic material may be appropriately blended with a viscosity adjusting component (e.g., resin or the like), a dispersion aid, a filler, etc. The hydrophobic material and the colorant are as described above with reference to the test chip.

The above-mentioned hydrophobic material is preferably heated and melted in forming the films. The heating temperature may be appropriately set in consideration of the melting point of the hydrophobic material and the viscosity adjusting component. In addition, the viscosity of the hydrophobic material at the time of melting may be appropriately selected so that the sheet-like material can be impregnated as desired, in consideration of the thickness and basis weight (density) of the sheet-like material to be used later.

The viscosity of the hydrophobic material is not particularly limited; however, from the viewpoint of sufficiently avoiding problems such as blockage during impregnation, the viscosity at 140°C and a shear rate of 3000 s-1 is preferably 100 mPa·s or less. , 50 mPa·s or less, more preferably 30 mPa·s or less.

The first substrate and the second substrate are not the constituent members of the finally obtained test chip, but one of the members used for manufacturing the test chip. As the first substrate and the second substrate, for example, there may be used a film made of polyesters, such as polyethylene terephthalate (PET) and polyethylene naphthalate (PEN), as well as polyphenylene sulfide (PPS), cellophane or the like. The first substrate and the second substrate may have any shape such as a ribbon shape and a film shape. Further, the same substrate may be used as the first substrate and the second substrate.

In the film forming step, the hydrophobic film may be formed on the first substrate and the second substrate by applying the above-mentioned hydrophobic material. At the time of coating, it is preferred to heat and hold the first substrate and the second substrate in advance. The thickness of the first hydrophobic film and the second hydrophobic film to be formed may be appropriately selected, in consideration of the thickness of the sheet-like material to be used later.

In particular, the ratio of the thickness of the second hydrophobic membrane (t2') to the thickness of the first hydrophobic membrane (t1') (thickness of the second hydrophobic film / thickness of the first hydrophobic film), that is, the ratio t2'/ t1'is preferably 0.56 or more and 2.2 or less. By setting the ratio t2'/t1' to 0.56 or more and 2.2 or less, problems such as blockage when impregnating the sheet-like material with the hydrophobic material in the subsequent process are sufficiently avoided, and the speed and/or speed stability of the liquid flow from the liquid receiving section A to the detection confirmation section B can be sufficiently increased. From the same viewpoint, the t2'/t1' is more preferably more than 1.0, further preferably 1.3 or more, and even more preferably 1.8 or more. However, the ratio t2'/t1' is not particularly limited and may be 3.0 or less.

### <First and second printing steps>

In the first printing step, the first hydrophobic film formed on the first substrate is used for carrying out printing on a first sacrificial substrate. Further, in the second printing step, the second hydrophobic film on the second substrate is used for carrying out printing on a second sacrificial substrate.

The first sacrificial substrate and the second sacrificial substrate are not the constituent members of the finally obtained test chip, but one of the members used for manufacturing the test chip. The first sacrificial substrate and the second sacrificial substrate are preferably for general-purposes, with which printing may be carried out with high accuracy, and may be high-quality paper, coated paper, synthetic paper, etc. The first sacrificial substrate and the second sacrificial substrate may be comprised of the same base material.

The printing on the first sacrificial substrate and the printing on the second sacrificial substrate are not particularly limited; for example, there may be preferably used a label writer widely used as office supplies. Then, in the first printing step, the first hydrophobic film on the first substrate is used to vary out printing on the first sacrificial substrate so as to have an inverted pattern of the first layer of the test chip. Further, in the second printing step, the second hydrophobic film on the second substrate is used to carry out printing on the second sacrificial substrate so as to have an inverted pattern of the second layer of the test chip. In this regard, taking the production of the test chip illustrated in FIG. 2 by way of example, the pattern printed on the first sacrificial substrate is a pattern having a printed film corresponding to the liquid receiving section A and the detection confirmation section B. Further, the pattern printed on the second sacrificial substrate is a pattern having a printed film corresponding to the liquid flow section C, the liquid flow section D, and the liquid passage E.

The pattern to be printed may be generated in advance by, for example, a personal computer or the like, and the data may be imported to the printing device. Further, the first printing step and the second printing step may be carried out simultaneously.

### <First and second transfer steps>

In the first transfer step, the first hydrophobic film after the first printing step is transferred to one surface of a single sheet-like material, and impregnated into the sheet-like material. Further, in the second transfer step, the second hydrophobic film after the second printing step is transferred to the other surface of the single sheet-like material, and impregnated into the sheet-like material. By this, a first layer derived from the first hydrophobic membrane, as well as a second layer derived from the second hydrophobic membrane are formed.

A transfer device, such as laminator, may be used for the transfer of the hydrophobic films. Further, in the transfer of the first hydrophobic film on the first substrate and the transfer of the second hydrophobic film on the second substrate, it is preferred that the respective transfer positions are appropriately adjusted according to the pattern of the desired test chip.

The sheet-like material is as described above with respect to the material M.

The impregnation of the hydrophobic membrane into the sheet-like material may be achieved, for example, by heating. In this regard, for example, if a transfer device such as a heatable laminator is used, it is possible to simultaneously carry out both the transfer and the impregnation of the hydrophobic films.

Here, in the first transfer step and the second transfer step, at least a part of the first hydrophobic film transferred from the first substrate and the second hydrophobic film transferred from the second substrate by means of the above-mentioned impregnation is made to contact in the material M. In other words, in the region of the sheet-like material where the hydrophobic films are transferred to both sides as seen in the thickness direction, the hydrophobic films are impregnated over the entire thickness direction. On the other hand, in the region of the sheet-like material where the hydrophobic films are transferred only to one surface as seen in the thickness direction, the other surface is not impregnated. Such adjustment may be carried out, for example, by appropriately adjusting the viscosity of the above-mentioned hydrophobic material, the thickness of the sheet-like material, the thickness of the hydrophobic membrane, etc.

In the first transfer step and the second transfer step, the entirety of the transferred hydrophobic films may be impregnated in the sheet-like material. In that case, the thickness of the sheet-like material does not significantly change before and after the first transfer step and the second transfer step (there is however a possibility that the thickness may decrease due to the influence of compression by the laminator, or the like).

The first transfer step may be carried out before the second transfer step or after the second transfer step. Alternatively, the first transfer step and the second transfer step may be carried out collectively and simultaneously. In that case, the sheet-like material may be sandwiched between the first substrate and the second substrate so that the hydrophobic films are brought into contact with the sheet-like material.

If the conditions of the first transfer step and the second transfer step are the same, the thicknesses ratio (t2/t1) of the first layer and the second layer formed after impregnation is maintained at the thickness ratio of each hydrophobic film before impregnation.

After the first transfer step and the second transfer step, the first substrate and the second substrate may be peeled off, as appropriate. In this way, it is possible to finally obtain the test chip of the present embodiment.

In the method for manufacturing a test chip according to the present embodiment, by carrying out the predetermined printing step and transfer step described above, even if a sheet-like material with a relatively rough surface such as a commercially available filter paper is used, transfer defects and voids are less likely to occur, and test chips can be manufactured with higher accuracy.

Further, in the method for manufacturing a test chip according to the present embodiment, since the first layer and the second layer having a desired pattern can be formed without using a mold or the like, on-demand manufacturing is made possible.

Further, in the test chip manufacturing method according to the present embodiment, since the test chip are manufactured by forming the first layer and the second layer on both sides of one sheet-shaped material, various advantages can be achieved as compared, for example, to the case where the test chip is manufactured by using two sheet-shaped materials (or where a single sheet-shaped material is folded), such as (1) the labor and cost of lamination process (or folding process) can be saved; (2) the flow of the test liquid due to the capillary action between the first layer and the second layer of the obtained test chip is ensured; and (3) no jigs or the like for holding the laminated body (or the folded body) of the sheet-like material are required so that the disposal becomes easy.

### (Experiments)

Next, the present disclosure will be described in further detail with reference to Examples and Comparative Examples, though the present disclosure is not limited to the following Examples.

### (Experiment 1)

First of all, the inventors examined the effects of the pattern (passage structure) of the first layer and the second layer of the test chip on the color unevenness.

### <Manufacturing of test chips>

Paraffin wax as a hydrophobic material ("Paraffin Wax - 155" manufactured by Nippon Seiki Co., Ltd.) 48 parts by mass, synthetic wax as a hydrophobic material ("Diacarna^{®} 80" manufactured by Mitsubishi Chemical Corp.) 48 parts by mass, ethylenevinyl acetate copolymer resin ("Ultrasen^{®} 681" manufactured by Toso Corp.) 2 parts by mass, and carbon black as a coloring agent ("MA-100" manufactured by Mitsubishi Chemical Corp.) 2 parts by mass were blended and melt-mixed at 100°C. At that time, each component was dispersed using a sand mill. In this way, the hydrophobic material was been prepared.

The above-mentioned hydrophobic material and a substrate (polyester film "Lumirror^{®} #5A-F531" manufactured by Toray Ind. Inc., with one side subjected to heat-resistant treatment) have been placed on a hot plate maintained at 120°C, with the non-heat-resistant side upside. The above-mentioned hydrophobic material was maintained in a molten state at 120°C and then applied onto the substrate with a Mayer bar to have a thickness of about 6 to 12 µm, so as to form ribbon-shaped hydrophobic films (the first hydrophobic film and the second hydrophobic film).

Next, by using a label writer ("TEPURA SR750" by King Jim Corp.), the above-mentioned hydrophobic film was printed on a high-quality paper as the first sacrificial substrate so as to have a desired pattern prepared in advance on a personal computer. Similarly, the above-mentioned hydrophobic film was printed on the high-quality paper as the second sacrificial substrate so as to have a desired pattern prepared in advance on a personal computer. The above two patterns printed on wood-free paper correspond to the inverted patterns of the front surface (first layer) and the back surface (second layer) of the finally obtained test chip, respectively. The high-quality paper after the printing is not for use in a subsequent process.

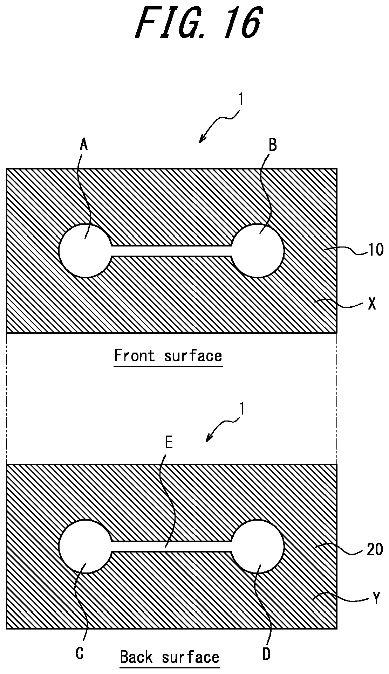

Here, in Comparative Example 1, the patterns on the front surface (the first layer) and the pattern on the back surface (the second layer) are as illustrated in FIG. 16. That is, in Comparative Example 1, the liquid receiving section A and the detection confirmation section B are connected by a liquid passage on the front surface, and the patterns of the front surface and the back surface are substantially the same. Further, in Examples 1-1 to 1-7, the pattern of the front surface (first layer) and the pattern of the back surface (second layer) are as illustrated in FIGS. 2, 9, 10, 11, 12, 13 and 14, respectively.

Next, by using the printed substrate, a filter paper (Whatman grade 41) as the sheet-shaped material M was sandwiched while carrying out appropriate positioning, and bringing the hydrophobic films into contact with the filter paper. Then, by using the laminator maintained at 90°C, the hydrophobic films were collectively transferred onto both sides of the filter paper. Each hydrophobic film was substantially completely impregnated into the filter paper from both sides, to make the portion of the filter paper directly underneath hydrophobic. By this, the first layer having a predetermined pattern was formed on the front surface side of the filter paper, and the second layer having the predetermined pattern was formed on the back surface side of the filter paper. In the filter paper, in the region where the hydrophobic films were transferred onto both sides in the thickness direction, the hydrophobic films were impregnated over the entire thickness direction. Further, in the region where the hydrophobic film was transferred onto only one surface in the thickness direction, the impregnation did not reach the other surface. Then, the substrates on both sides were peeled off, to finally obtain a test chip. Since the first layer and the second layer in each example are derived from the same hydrophobic film, they had the same thickness.

### <Evaluation of color development unevenness>

For each of the obtained test chips, the front surface (the first layer) was placed on the upper side, and the center of the detection confirmation section B was marked with a water-based red highlight pen. Then, three drops of distilled water were dropped into the liquid receiving portion A with a dropper, and the change of the red mark due to the flow of water was observed.

As a result, in Comparative Example 1 (FIG. 16), the red mark in the center of the detection confirmation section B moved toward the outer periphery of the detection confirmation section B (particularly, the side farther from the liquid receiving section A), and it was difficult to confirm the red color by visual observation. Thus, the test chip of Comparative Example 1 was recognized to have uneven color development, and is therefore unsuitable for quantitative analysis.

In contrast, in Examples 1-1 to 1-7 (FIGS. 2, 9 to 14), the highlight mark in the center of the detection confirmation section B remained inside the outer periphery of the detection confirmation section B. In particular, in Examples 1-2 to 1-7 (FIGS. 9 to 14), the highlighter mark in the center of the detection confirmation section B stayed closer to the center of the detection confirmation section B. It is considered that this is because the liquid passage is optimized so that water can flow three-dimensionally in the filter paper, as compared with Comparative Example 1. Thus, the test chips of Examples 1-1 to 1-7 were recognized to significantly suppress the color unevenness and, therefore, quantitative analysis can be expected.

### (Experiment 2)

Next, the inventors examined the relationship between the thicknesses of the first layer and the second layer, which can ensure good liquid flowability.

Hydrophobic materials (inks) were prepared according to the formulation as listed in Table 1.

**Table 1**

| | | Ink 1 | Ink 2 |
|---|---|---|---|
| Paraffin wax * 1 | Parts by mass | 72.0 | 72.0 |
| Synthetic wax * 2 | | 18.0 | 18.0 |
| Carbon black *3 | | 1.8 | 1.8 |
| Resin *4 | | 11.25 | 9.0 |
| Viscosity (140°C,3000/s) | mPa·s | 23 | 17 |
| Density | g/cm³ | 0.85 | 0.85 |

| | | | |
|---|---|---|---|
| * 1 "Paraffin Wax -135" by Nippon Seiro Co., Ltd., * 2 Synthetic wax "Diacarna^{®} 30", by Mitsubishi Chemical Corp. * 3 Carbon black "MA-100" by Mitsubishi Chemical Corp. * 4 Resin " Ultrasen^{®} 722 " by Tosoh Corp. | | | |

Test chips were obtained in essentially the same way as above, except that ink 1 or ink 2 was used as the hydrophobic material, Watman grade 41 (filter paper # 41) or Whatman grade 40 (filter paper # 40) was used as the filter paper (material M), and the temperature of the laminator was appropriately adjusted. At that time, the patterns of the first layer and the second layer were as illustrated in FIG. 11. At that time, the thickness of the first hydrophobic film for forming the first layer and the thickness of the second hydrophobic film for forming the second layer (corresponding to the thickness ratio of the first layer and the second layer) were changed as appropriate.

Each of the obtained test chips was placed, with the front surface (first layer) on the upper side. Next, about 0.3 mL of a liquid obtained by dissolving aqueous fluorescent ink in distilled water was dropped into the liquid receiving section A with a dropper, to measure the time (flow time) from the start of the dropping until the liquid reaches the detection confirmation section B. The same measurement was carried out eight times, for calculating the average value and the standard deviation. The results are listed in Tables 2 to 5 for each combination of the hydrophobic material and the filter paper.

**Table 2**

| (By us ing ink 1 and filter paper #41) | | Example 2-1 | Example 2-2 | Example 2-3 | Example 2-4 | Example 2-5 | Example 2-6 | Example 2-7 | Example 2-8 | Example 2-9 |
|---|---|---|---|---|---|---|---|---|---|---|
| Thickness of first hydrophobic film | [µm] | 6 | 6 | 6 | 6 | 6 | 10.1 | 10.1 | 10.8 | 10.8 |
| Thickness of second hydrophobic film | [µm] | 6 | 10.1 | 10.8 | 11.8 | 12.6 | 6 | 10.1 | 6 | 10.8 |
| Total thickness of hydrophobic films | [µm] | 12 (min) | 16.1 | 16.8 | 17.8 | 18.6 | 16.1 | 20.2 | 16.8 | 21.6 (max) |
| Thickness of second hydrophobic film / thickness of first hydrophobic film | - | 1.0 | 1.7 | 1.8 | 2.0 | 2.1 | 0.59 | 1.0 | 0.56 | 1.0 |
| Average flow time | [sec] | 56.35 | 55.72 | 49.13 | 46.56 | 52.23 | 66.78 | 41.12 | 53.69 | 50.17 |
| Standard deviation of flow time | - | 11.89 | 8.46 | 6.10 | 4.94 | 3.58 | 13.77 | 3.63 | 13.04 | 4.78 |

**Table 3**

| (By using ink 2 and filter paper #41) | | Example 3-1 | Example 3-2 | Example 3-3 | Example 3-4 | Example 3-5 | Example 3-6 |
|---|---|---|---|---|---|---|---|
| Thickness of first hydrophobic film | [µm] | 5.4 | 5.4 | 5.4 | 5.4 | 9.5 | 10 |
| Thickness of second hydrophobic film | [µm] | 9.5 | 10 | 11.5 | 11.8 | 9.5 | 10 |
| Total thickness of hydrophobic films | [µm] | 14.9 (min) | 15.4 | 16.9 | 17.2 | 19 | 20 (max) |
| Thickness of second hydrophobic film / thickness of first hydrophobic film | - | 1.8 | 1.9 | 2.1 | 2.2 | 1.0 | 1.0 |
| Average flow time | [sec] | 43.81 | 59.54 | 52.04 | 52.67 | 44.70 | 47.07 |
| Standard deviation of flow time | - | 5.64 | 6.74 | 4.23 | 5.66 | 4.34 | 4.52 |

**Table 4**

| (By using ink 1 and filter paper #40) | | Example 4-1 | Example 4-2 | Example 4-3 | Example 4-4 |
|---|---|---|---|---|---|
| Thickness of first hydrophobic film | [µm] | 6 | 6 | 10.1 | 10.8 |
| Thickness of second hydrophobic film | [µm] | 10.1 | 10.8 | 10.1 | 10.8 |
| Total thickness of hydrophobic films | [µm] | 16.1 (min) | 16.8 | 20.2 | 21.6 (max) |
| Thickness of second hydrophobic film / thickness of first hydrophobic film | - | 1.7 | 1.8 | 1.0 | 1.0 |
| Average flow time | [sec] | 221.00 | 221.13 | 241.13 | 163.00 |
| Standard deviation of flow time | - | 30.39 | 27.90 | 25.80 | 9.13 |

**Table 5**

| (By using ink 2 and filter paper #40)) | | Example 5-1 | Example 5-2 | Example 5-3 | Example 5-4 | Example 5-5 |
|---|---|---|---|---|---|---|
| Thickness of first hydrophobic film | [µm] | 5.4 | 5.4 | 9.5 | 9.5 | 10 |
| Thickness of second hydrophobic film | [µm] | 11.5 | 11.8 | 5.4 | 9.5 | 10 |
| Total thickness of hydrophobic films | [µm] | 16.9 | 17.2 | 14.9 (min) | 19 | 20 (max) |
| Thickness of second hydrophobic film/ thickness of first hydrophobic film | - | 2.1 | 2.2 | 0.57 | 1.0 | 1.0 |
| Average flow time | [sec] | 242.13 | 224.38 | 282.00 | 229.38 | 260.50 |
| Standard deviation of flow time | - | 18.27 | 18.61 | 43.64 | 14.60 | 53.34 |

It may be concluded that all of the examples in Table 2 and Table 5 have good liquid flowability. From this, as regards the ratio of the thickness of the second layer to the thickness of the first layer (the thickness of the second layer / the thickness of the first layer), as long as the ratio is within the range of at least 0.56 to 2.2, a good liquid flowability can be guaranteed.

### (Experiment 3)

Next, the inventors examined the impregnation rate of the hydrophobic material that can ensure good liquid flowability.

The same filter paper (the filter paper # 41 or the filter paper # 40) as used in Experiment 2 was cut into a size of 5 cm × 2 cm, and dried at 120°C for 3 minutes, to measure the dry mass M0 (g). Then, the filter paper was immersed in the same hydrophobic material (ink 1 or ink 2) as that used in Experiment 2, and left at 120°C for 3 minutes. The filter paper after immersion was sandwiched between the same type of filter paper and slide glass, and left at 120 °C. for 1 minute under a load of 100 gf to remove excess hydrophobic material. The mass M1 (g) of the filter paper was then measured. The maximum impregnation amount Pmax (g/m²) per unit section area was then calculated from (M1-M0) × 1000.

In each of Tables 2 to 5, examples with the maximum and minimum total thickness of the first hydrophobic film and the second hydrophobic film were selected, respectively, and the ink densities listed in Table 1 are used, to calculate the actual impregnation amount P (g/m²) per unit section area in the selected example. Then, the impregnation rate (%) of the hydrophobic material in the filter paper was calculated as (P/Pmax) × 100. The results are listed in Table 6.

**Table 6**

| Ink type | | Ink 1 | | Ink 2 | | Ink 1 | | Ink 2 | |
|---|---|---|---|---|---|---|---|---|---|
| Filter paper type | | Filter paper #41 | | Filter paper #41 | | Filter paper #40 | | Filter paper #40 | |
| Maximum impregnation amount (Pmax) | [g/m²] | 68.30 | | 70.10 | | 60.80 | | 55.43 | |

| | | Example 2-1 | Example 2-9 | Example 3-1 | Example 3-6 | Example 4-1 | Example 4-4 | Example 5-3 | Example 5-5 |
|---|---|---|---|---|---|---|---|---|---|
| Total thickness of hydrophobic films | [µm] | 12 (min) | 21.6 (max) | 14.9 (min) | 20 (max) | 16.1 (min) | 21.6 (max) | 14.9 (min) | 20 (max) |
| Actual impregnation amount (P) | [g/m²] | 10.2 | 18.4 | 12.7 | 17.0 | 13.7 | 18.4 | 12.7 | 17.0 |
| Impregnation rate of hydrophobic material | [%] | 14.9 | 26.9 | 18.1 | 24.3 | 22.5 | 30.2 | 22.8 | 30.7 |

From Table 6, it may be considered that good liquid flowability can be ensured if the impregnation rate of the hydrophobic material is at least in the range of about 14% to 32%.

### INDUSTRIAL APPLICABILITY

The present disclosure provides a test chip wherein the target substance contained in the test liquid is reacted with the pre-charged labeling medium for confirming the presence of the target substance by the reaction between, and wherein uneven color development is significantly suppressed. Further, the present disclosure also provides a method for manufacturing a test chip, with which the above-mentioned test chip can be easily manufactured with high accuracy and at low cost.

### (Reference numerals)

- 1: Test chip
- 10: First layer
- 10': Pre-first layer
- 20: Second layer
- 20': Pre-second layer
- 50: Hydrophobic material
- A: Liquid receiving section
- B: Detection confirmation section
- C, D: Liquid flow section
- D': Liquid non-flow section
- E, E1, E2, E3, E4, E31, E32: Liquid passage
- F: Liquid passage
- M: Material that permits flow of the test liquid
- M': Material that prohibits flow of the test liquid
- X, Y: Liquid non-flow section

## Claims

1. A sheet-shaped test chip (1) comprising:
a first layer (10) on a front surface side and a second layer (20) on a back surface side;
wherein the first layer (10) and the second layer (20) are adjacent to each other;
wherein one of the first layer (10) and the second layer (20) has a liquid receiving section A;
wherein the first layer (10) has at least a detection confirmation section B;
wherein the second layer (20) has at least a liquid flow section D adjacent to the detection confirmation section B, and a liquid passage E connected to the liquid flow section D;
wherein, in case where the liquid receiving section A is provided in the first layer (10), the liquid receiving section A is spaced from the detection confirmation section B; and
wherein, when a sample test liquid is dropped into the liquid receiving section A, the test liquid passes through the liquid receiving section A, the liquid passage E, and the liquid flow section D in the stated order, by means of capillary action, and flows to the detection confirmation section B;
**characterized in that** the liquid flow section D has an annular structure formed with a liquid non-flow section D' therein.

2. The test chip (1) according to claim 1, wherein the second layer (20) is provided with a plurality of the liquid passages E.

3. The test chip (1) according to claim 2, wherein at least two of the liquid passages E are connected to the liquid flow section D so as to face each other.

4. The test chip (1) according to claim 2 or 3, wherein at least two of the liquid passages E have substantially the same shape.

5. The test chip (1) according to any one of claims 1 to 4, wherein:
the first layer (10) comprises the liquid receiving section A that is spaced from the detection confirmation section B, the second layer (20) comprises the liquid flow section C adjacent to the liquid receiving section A; and
the test chip (1) is configured such that, when the sample test liquid is dropped into the liquid receiving section A, the test liquid passes through the liquid receiving section A, the liquid flow section C, the liquid passage E, and the liquid flow section D in this order, due to a capillary action and flows to the detection confirmation section B.

6. The test chip (1) according to any one of claims 1 to 4, wherein:
the first layer (10) comprises the liquid receiving section A that is spaced from the detection confirmation section B, and the liquid passage F that is connected to the liquid receiving section A; and
the test chip (1) is configured so that, when the test liquid is dropped into the liquid receiving section A, the test liquid passes through the liquid receiving portion A, the liquid passage F, the liquid passage E, and the liquid flow section D in this order, due to the capillary action and flows to the detection confirmation section B.

7. The test chip (1) according to any one of claims 1 to 4, wherein the second layer (20) comprises the liquid receiving portion A.

8. The test chip (1) according to any one of claims 1 to 7, wherein:
the liquid receiving section A, any liquid flow section C, any liquid passage F, the liquid passage E, the liquid flow section D, and the detection confirmation section B, are made of a material M that permits flow of the test liquid by means of capillary action; and
the part other than the material M is made of a material M' in which the material M is impregnated with the hydrophobic material that prohibits flow of the test liquid.

9. The test chip (1) according to claim 8, wherein the material M is a filter paper.

10. The test chip (1) according to claim 8 or 9 wherein, in the material M', an impregnation rate of the hydrophobic material into the material M is 14% or more and 32% or less.

11. The test chip (1) according to any one of claims 1 to 10, wherein a ratio of the thickness of the second layer (20) to the thickness of the first layer (10) (thickness of the second layer (20) / thickness of the first layer (10)) is 0.56 or more and 2.2 or less.

12. The test chip (1) according to any one of claims 1 to 11, being provided with a medium that reacts with a substance to be detected and/or a labeling medium adapted to cause a color reaction due to the substance to be detected so that a color development reaction due to a substance to be detected takes place in the detection confirmation section B.

13. A method for manufacturing a test chip (1) according to any one of claims 1 to 12, comprising:
a film formation step wherein a hydrophobic material is used for forming a first hydrophobic film on a first substrate, and a second hydrophobic film on a second substrate;
a first printing step wherein the first hydrophobic film on the first substrate is used for carrying out printing on a first sacrificial substrate, so as to be of an inverted pattern of a first layer (10) of the test chip (1);
a second printing process wherein the second hydrophobic film on the second substrate is used for carrying out printing on a second sacrificial substrate, so as to be of an inverted pattern of the second layer (20) of the test chip (1);
a first transfer step wherein the first hydrophobic film after the first printing step is transferred onto one surface of a single sheet-like material and impregnated into the sheet-like material; and
a second transfer step wherein the second hydrophobic film after the second printing step is transferred onto the other surface of the single sheet-like material and impregnated into the sheet-like material.

14. The method for manufacturing a test chip (1) according to claim 13, wherein a ratio of the thickness of the second hydrophobic membrane to the thickness of the first hydrophobic membrane (thickness of the second hydrophobic membrane / thickness of the first hydrophobic membrane) is 0.56 or more and 2.2 or less.

## Patentansprüche

1. Folienförmiger Prüfchip (1), umfassend:
eine erste Schicht (10) auf einer vorderen Oberflächenseite und eine zweite Schicht (20) auf einer hinteren Oberflächenseite;
wobei die erste Schicht (10) und die zweite Schicht (20) aneinander angrenzend sind;
wobei eine aus der ersten Schicht (10) und der zweiten Schicht (20) einen Flüssigkeitsaufnahmeabschnitt A aufweist;
wobei die erste Schicht (10) mindestens einen Nachweisbestätigungsabschnitt B aufweist;
wobei die zweite Schicht (20) mindestens einen Flüssigkeitsfließabschnitt D angrenzend an den Nachweisbestätigungsabschnitt B und einen mit dem Flüssigkeitsfließabschnitt D verbundenen Flüssigkeitsdurchgang E aufweist;
wobei, in einem Fall, wo der Flüssigkeitsaufnahmeabschnitt A in der ersten Schicht (10) bereitgestellt ist, der Flüssigkeitsaufnahmeabschnitt A von dem Nachweisbestätigungsabschnitt B beabstandet ist; und
wobei, wenn eine Probenprüfflüssigkeit in den Flüssigkeitsaufnahmeabschnitt A getropft wird, die Prüfflüssigkeit durch den Flüssigkeitsaufnahmeabschnitt A, den Flüssigkeitsdurchgang E und den Flüssigkeitsfließabschnitt D in der aufgeführten Reihenfolge mittels einer Kapillarwirkung hindurchgelangt und zum Nachweisbestätigungsabschnitt B fließt;
**gekennzeichnet dadurch, dass** der Flüssigkeitsfließabschnitt D eine ringförmige Struktur aufweist, die mit einem Flüssigkeits-Nichtfließabschnitt D' in derselben ausgebildet ist.

2. Prüfchip (1) nach Anspruch 1, wobei die zweite Schicht (20) mit einer Vielzahl der Flüssigkeitsdurchgänge E versehen ist.

3. Prüfchip (1) nach Anspruch 2, wobei mindestens zwei der Flüssigkeitsdurchgänge E mit dem Flüssigkeitsfließabschnitt D so verbunden sind, dass sie einander zugewandt sind.

4. Prüfchip (1) nach Anspruch 2 oder 3, wobei mindestens zwei der Flüssigkeitsdurchgänge E im Wesentlichen dieselbe Form aufweisen.

5. Prüfchip (1) nach einem der Ansprüche 1 bis 4, wobei:
die erste Schicht (10) den Flüssigkeitsaufnahmeabschnitt A umfasst, der von dem Nachweisbestätigungsabschnitt B beabstandet ist, die zweite Schicht (20) den Flüssigkeitsfließabschnitt C angrenzend an den Flüssigkeitsaufnahmeabschnitt A umfasst; und
der Prüfchip (1) so konfiguriert ist, dass, wenn die Probenprüfflüssigkeit in den Flüssigkeitsaufnahmeabschnitt A getropft wird, die Prüfflüssigkeit durch den Flüssigkeitsaufnahmeabschnitt A, den Flüssigkeitsfließabschnitt C, den Flüssigkeitsdurchgang E und den Flüssigkeitsfließabschnitt D in dieser Reihenfolge aufgrund einer Kapillarwirkung hindurchgelangt und zum Nachweisbestätigungsabschnitt B fließt.

6. Prüfchip (1) nach einem der Ansprüche 1 bis 4, wobei:
die erste Schicht (10) den Flüssigkeitsaufnahmeabschnitt A, der von dem Nachweisbestätigungsabschnitt B beabstandet ist, und den Flüssigkeitsdurchgang F, der mit dem Flüssigkeitsaufnahmeabschnitt A verbunden ist, umfasst; und
der Prüfchip (1) so konfiguriert ist, dass, wenn die Probenprüfflüssigkeit in den Flüssigkeitsaufnahmeabschnitt A getropft wird, die Prüfflüssigkeit durch den Flüssigkeitsaufnahmeteil A, den Flüssigkeitsdurchgang F, den Flüssigkeitsdurchgang E und den Flüssigkeitsfließabschnitt D in dieser Reihenfolge aufgrund der Kapillarwirkung hindurchgelangt und zum Nachweisbestätigungsabschnitt B fließt.

7. Prüfchip (1) nach einem der Ansprüche 1 bis 4, wobei die zweite Schicht (20) den Flüssigkeitsaufnahmeteil A umfasst.

8. Prüfchip (1) nach einem der Ansprüche 1 bis 7, wobei:
der Flüssigkeitsaufnahmeabschnitt A, alle Flüssigkeitsfließabschnitte C, alle Flüssigkeitsdurchgänge F, der Flüssigkeitsdurchgang E, der Flüssigkeitsfließabschnitt D und der Nachweisbestätigungsabschnitt B aus einem Material M hergestellt sind, das den Fluss der Prüfflüssigkeit mittels Kapillarwirkung ermöglicht; und
der vom Material M verschiedene Teil aus einem Material M' hergestellt ist, in dem das Material M mit dem hydrophoben Material imprägniert ist, das einen Fluss der Prüfflüssigkeit verhindert.

9. Prüfchip (1) nach Anspruch 8, wobei das Material M ein Filterpapier ist.

10. Prüfchip (1) nach Anspruch 8 oder 9, wobei in dem Material M' eine Imprägnierungsrate des hydrophoben Materials in das Material M 14 % oder mehr und 32 % oder weniger beträgt.

11. Prüfchip (1) nach einem der Ansprüche 1 bis 10, wobei ein Verhältnis der Dicke der zweiten Schicht (20) zu der Dicke der ersten Schicht (10) (Dicke der zweiten Schicht (20) / Dicke der ersten Schicht (10)) 0,56 oder mehr und 2,2 oder weniger beträgt.

12. Prüfchip (1) nach einem der Ansprüche 1 bis 11, der mit einem Medium, das mit einer nachzuweisenden Substanz reagiert, und/oder mit einem Markierungsmedium versehen ist, das dazu angepasst ist, eine Farbreaktion aufgrund der nachzuweisenden Substanz auszulösen, sodass eine Farbentwicklungsreaktion aufgrund einer nachzuweisenden Substanz in dem Nachweisbestätigungsabschnitt B erfolgt.

13. Verfahren zum Herstellen eines Prüfchips (1) nach einem der Ansprüche 1 bis 12, umfassend:
einen Filmausbildungsschritt, wobei ein hydrophobes Material zum Ausbilden eines ersten hydrophoben Films auf einem ersten Substrat und eines zweiten hydrophoben Films auf einem zweiten Substrat verwendet wird;
einen ersten Druckschritt, wobei der erste hydrophobe Film auf dem ersten Substrat zum Durchführen des Druckens auf einem ersten Opfersubstrat verwendet wird, sodass es ein umgekehrtes Muster einer ersten Schicht (10) des Prüfchips (1) ist;
einen zweiten Druckprozess, wobei der zweite hydrophobe Film auf dem zweiten Substrat zum Durchführen des Druckens auf einem zweiten Opfersubstrat verwendet wird, sodass es ein umgekehrtes Muster einer zweiten Schicht (20) des Prüfchips (1) ist;
einen ersten Übertragungsschritt, wobei der erste hydrophobe Film nach dem ersten Druckschritt auf eine Oberfläche eines einzelnen folienähnlichen Materials übertragen und in das folienähnliche Material imprägniert wird; und
einen zweiten Übertragungsschritt, wobei der zweite hydrophobe Film nach dem zweiten Druckschritt auf die andere Oberfläche des einzelnen folienähnlichen Materials übertragen und in das folienähnliche Material imprägniert wird.

14. Verfahren zum Herstellen eines Prüfchips (1) nach Anspruch 13, wobei ein Verhältnis der Dicke der zweiten hydrophoben Membran zu der Dicke der ersten hydrophoben Membran (Dicke der zweiten hydrophoben Membran / Dicke der ersten hydrophoben Membran) 0,56 oder mehr und 2,2 oder weniger beträgt.

## Revendications

1. Puce de test (1) en forme de feuille comprenant :
une première couche (10) sur un côté de surface avant et une deuxième couche (20) sur un côté de surface arrière ;
dans laquelle la première couche (10) et la deuxième couche (20) sont adjacentes l'une à l'autre ;
dans laquelle l'une de la première couche (10) et de la deuxième couche (20) présente une section de réception de liquide A ;
dans laquelle la première couche (10) présente au moins une section de confirmation de détection B ;
dans laquelle la deuxième couche (20) présente au moins une section d'écoulement de liquide D adjacente à la section de confirmation de détection B, et un passage de liquide E relié à la section d'écoulement de liquide D ;
dans laquelle, au cas où la section de réception de liquide A est pourvue dans la première couche (10), la section de réception de liquide A est espacée de la section de confirmation de détection B ; et
dans laquelle, lorsqu'un échantillon de liquide de test tombe dans la section de réception de liquide A, le liquide de test passe à travers la section de réception de liquide A, le passage de liquide E et la section d'écoulement de liquide D dans l'ordre indiqué, au moyen d'une action capillaire, et s'écoule vers la section de confirmation de détection B ;
**caractérisée en ce que** la section d'écoulement de liquide D a une structure annulaire formée d'une section sans écoulement de liquide D' à l'intérieur de celle-ci.

2. Puce de test (1) selon la revendication 1, dans laquelle la deuxième couche (20) est pourvue d'une pluralité de passages de liquide E.

3. Puce de test (1) selon la revendication 2, dans laquelle au moins deux des passages de liquide E sont reliés à la section d'écoulement de liquide D de manière à se faire face.

4. Puce de test (1) selon la revendication 2 ou 3, dans laquelle au moins deux des passages de liquide E ont sensiblement la même forme.

5. Puce de test (1) selon l'une quelconque des revendications 1 à 4, dans laquelle :
la première couche (10) comprend la section de réception de liquide A qui est espacée de la section de confirmation de détection B, la deuxième couche (20) comprend la section d'écoulement de liquide C adjacente à la section de réception de liquide A ; et
la puce de test (1) est configurée de telle sorte que, lorsque l'échantillon de liquide de test tombe dans la section de réception de liquide A, le liquide de test passe à travers la section de réception de liquide A, la section d'écoulement de liquide C, le passage de liquide E et la section d'écoulement de liquide D dans cet ordre, en raison d'une action capillaire et s'écoule vers la section de confirmation de détection B.

6. Puce de test (1) selon l'une quelconque des revendications 1 à 4, dans laquelle :
la première couche (10) comprend la section de réception de liquide A qui est espacée de la section de confirmation de détection B, et le passage de liquide F qui est relié à la section de réception de liquide A ; et
la puce de test (1) est configurée de telle sorte que, lorsque le liquide de test tombe dans la section de réception de liquide A, le liquide de test passe à travers la portion de réception de liquide A, le passage de liquide F, le passage de liquide E et la section d'écoulement de liquide D dans cet ordre, en raison d'une action capillaire et s'écoule vers la section de confirmation de détection B.

7. Puce de test (1) selon l'une quelconque des revendications 1 à 4, dans laquelle la deuxième couche (20) comprend la portion de réception de liquide A.

8. Puce de test (1) selon l'une quelconque des revendications 1 à 7, dans laquelle :
la section de réception de liquide A, toute section d'écoulement de liquide C, tout passage de liquide F, le passage de liquide E, la section d'écoulement de liquide D et la section de confirmation de détection B sont constitués d'un matériau M qui permet l'écoulement du liquide de test au moyen d'une action capillaire ; et
la partie autre que le matériau M est constituée d'un matériau M' dans lequel le matériau M est imprégné du matériau hydrophobe qui empêche l'écoulement du liquide de test.

9. Puce de test (1) selon la revendication 8, dans laquelle le matériau M est un papier filtre.

10. Puce de test (1) selon la revendication 8 ou 9, dans laquelle, dans le matériau M', un taux d'imprégnation du matériau hydrophobe dans le matériau M est de 14 % ou plus et de 32 % ou moins.

11. Puce de test (1) selon l'une quelconque des revendications 1 à 10, dans laquelle le rapport de l'épaisseur de la deuxième couche (20) sur l'épaisseur de la première couche (10) (épaisseur de la deuxième couche (20) / épaisseur de la première couche (10)) est de 0,56 ou plus et de 2,2 ou moins.

12. Puce de test (1) selon l'une quelconque des revendications 1 à 11, étant pourvue d'un milieu qui réagit avec une substance à détecter et/ou d'un milieu de marquage adapté pour provoquer une réaction colorée due à la substance à détecter de sorte qu'une réaction de développement de couleur due à une substance à détecter a lieu dans la section de confirmation de détection B.

13. Procédé de fabrication d'une puce de test (1) selon l'une quelconque des revendications 1 à 12, comprenant :
une étape de formation de film dans laquelle un matériau hydrophobe est utilisé pour former un premier film hydrophobe sur un premier substrat, et un deuxième film hydrophobe sur un deuxième substrat ;
une première étape d'impression dans laquelle le premier film hydrophobe sur le premier substrat est utilisé pour réaliser une impression sur un premier substrat sacrificiel, de manière à constituer un motif inversé d'une première couche (10) de la puce de test (1) ;
un deuxième processus d'impression dans lequel le deuxième film hydrophobe sur le deuxième substrat est utilisé pour réaliser une impression sur un deuxième substrat sacrificiel, de manière à constituer un motif inversé de la deuxième couche (20) de la puce de test (1) ;
une première étape de transfert dans laquelle le premier film hydrophobe après la première étape d'impression est transféré sur une surface d'un matériau de type feuille unique et imprégné dans le matériau de type feuille ; et
une deuxième étape de transfert dans laquelle le deuxième film hydrophobe après la deuxième étape d'impression est transféré sur l'autre surface du matériau de type feuille unique et imprégné dans le matériau de type feuille.

14. Procédé de fabrication d'une puce de test (1) selon la revendication 13, dans lequel le rapport de l'épaisseur de la deuxième membrane hydrophobe sur l'épaisseur de la première membrane hydrophobe (épaisseur de la deuxième membrane hydrophobe / épaisseur de la première membrane hydrophobe) est de 0,56 ou plus et de 2,2 ou moins.
